# EUROPEAN PATENT APPLICATION

(11) **EP 1 083 428 A2**
(43) Date of publication of application: **14.03.2001**
(21) Application number: 00117300.4
(22) Date of filing: 18.08.2000
(51) Int. Cl.: G01N 33/576, G01N 33/58, C07K 16/10, G01N 33/53

(54) **Method and reagent for the detection or determination of HCV core antigens**

(30) Priority: 19.08.1999 JP 23317199
(71) Applicant: KYOWA MEDEX CO., LTD., Tokyo 104-0042 (JP)
(72) Inventor: Morita, Kazuki, Kyowa Medex R&L., Kyowa Medex Ltd, Sunto-gun, Shizuoka 411-0932 (JP); Nishida, Ayako, Kyowa Medex R&L., Kyowa Medex Ltd, Sunto-gun, Shizuoka 411-0932 (JP); Fukui, Masanori, Kyowa Medex Co. Ltd., Tokyo 104-0042 (JP); Kondou, Masaru, Kyowa Medex Co. Ltd., Tokyo 104-0042 (JP); Kohara, Michinori, Tokyo 114-0014 (JP)
(74) Representative: Casalonga, Axel

(57) **Abstract**

The present invention provides an immunoassay for the detection of HCV core antigens in a sample which comprises subjecting an alkaline phosphatase-labeled anti-HCV core protein antibody and an HCV core antigen in the sample to antigen-antibody reaction in an aqueous solution containing a salt at a high concentration to form an antigen-antibody complex, and measuring the alkaline phosphatase activity of the formed antigen-antibody complex. Also provided is a reagent for the detection of HCV core antigens comprising an alkaline phosphatase-labeled anti-HCV core protein antibody, a salt at a concentration of 0.5 M to saturation and a reagent for the measurement of alkaline phosphatase activity.

## Description

### Background of the Invention

The present invention relates to a method for the detection or determination of hepatitis C virus (HCV) and a reagent for the detection or determination of HCV.

Among the methods used for the test for a causative virus of infection are antibody test, antigen test and genetic test.

Immunoassay is the principal method for the antibody test and the antigen test and is widely employed for the diagnosis of viral infection, virus screening tests of blood for transfusion, monitoring for the treatment of viral infection, etc. The genetic test can be carried out by using gene amplification methods such as polymerase chain reaction (PCR), ligase chain reaction (LCR) and nucleic acid sequence based amplification (NASBA), the branched DNA (bDNA) method with an improved labeling efficiency, etc. and has enabled measurements with high sensitivity.

The genetic test is used not only for definite diagnosis, but also for the detection of virus at levels below the detection limits of the above-mentioned antibody test and antigen test and for the evaluation of virus elimination in medical treatments. Recently, the gene amplification methods are expected to be applicable to screening tests of blood for transfusion to detect viral infection in the early stage that is incapable of being detected by the antibody test or antigen test.

However, the genetic test has various problems; for example, the procedure is complicated and a special apparatus is necessary, technical skills are required, the cost is high, the reproducibility and measurement precision are not sufficient, it is not suitable for the treatment of a lot of samples, it is liable to show false negative results due to inhibition factors in samples, a nucleic acid itself is unstable as compared with a protein in samples, and the amplification efficiency of a gene is not constant. It is reported that when a virus-derived nucleic acid is RNA, the nucleic acid is still less stable and gives results widely varying depending upon the test process and the conditions of preservation of test samples.

The test for an antibody to a virus, which detects an antibody to a virus produced by the in vivo immune mechanism, is a method of observing the effect of viral infection. Antibody production against an antigen differs among individuals; that is, there exist individuals showing a high antibody titer and those not capable of producing the antibody at all. Further, it is difficult to predict the actual conditions of a patient and the amount of virus in the body of a patient because the amount of antibody do not necessarily correspond to the amount of virus. There is still the problem that an accurate judgment can not be made because antibodies are not produced in the early stage of viral infection, which makes it impossible to detect infection in the early stage, and non-specific reactions of antibodies are also observed.

The antigen test is a method of detecting a virus-derived antigen and various assay systems for virus-derived antigens are commercially available. For the antigen test, immunoassay using an antibody which recognizes a virus-derived antigen is mainly employed, but it is insufficient in sensitivity as compared with the gene amplification methods. Accordingly, there remains the problem that a small amount of virus can not be detected accurately. Further, a virus and antibodies to the virus coexist in vivo, and these coexisting antibodies and the antibody used in immunoassay react competitively with the antigen, which sometimes makes it difficult to assay the antigen with a high sensitivity. There have been developed antigen assay systems for the detection of HBV and other hepatitis viruses, HIV, etc., but they are less sensitive than the DNA amplification methods such as PCR and are not sufficient for the use as a marker for diagnosis or therapy.

Recently, an assay system for HCV core protein which is the internal antigen of HCV has been developed (Japanese Published Unexamined Patent Application No. 29427/96). This method is simple and capable of stable measurement as compared with the methods in which genes are assayed, and the kit employed in the method is applicable to the definite diagnosis of HCV and the prediction and judgment of efficacy of therapy of HCV. However, this method is insufficient in sensitivity as compared with the method by PCR, and so this HCV core protein assay alone is not sufficient for the use as a marker for diagnosis or therapy. Actually, the limit for detection of this method is 8 pg/ml (10⁴⁻⁵ virions/ml in PCR titer), which is 10 or more times higher that that of the method by PCR. Accordingly, in judging the efficacy of a therapy, samples whose HCV core protein content is 8 pg/ml or lower are judged to be negative, which means that the method is incapable of detecting a small amount of virus.

It is generally known that the use of a salt at high concentrations in antigen-antibody reactions inhibits the reactions and dissociates antigens from the antigen-antibody reaction products.

For example, there is the following description in the 9th chapter of Supersensitive Enzyme Immunoassay [Eiji Ishikawa, Gakkai Shuppan Center (1993)]: "As the degree of non-specific adsorption of a protein to a solid phase varies according to conditions, it is expected that the serum interference can be reduced by adjusting conditions so as to lower the degree of non-specific adsorption. In the sandwich immunoassay of insulin, the serum interference is reduced by lowering the concentration of serum in the incubation with anti-insulin IgG-immobilized polystyrene balls, and by reducing the incubation time (Table IX-14) (Ruan, et al., 1986) . The serum interference can also be reduced by raising the concentration of an inorganic salt to lower the degree of non-specific adsorption of a protein to a solid phase (Table IX-15, Figs. IX-18-IX-21) (Hashida, et al., 1983). The effective concentration varies depending upon the kind of inorganic salt; however, the immune reaction itself is inhibited if the concentration is too high. For example, in the case of sodium chloride, the maximum concentration may be 0.3-0.4 mol/l."

An object of the present invention is to provide a method for detecting or determining HCV antigens which is capable of detecting or determining HCV antigens simply and with a high sensitivity and which is suitable for use in screenings in blood business and health examinations where a large number of samples are treated, and a reagent for the detection or determination of HCV antigens for use therein.

### Summary of the Invention

The present inventors have unexpectedly found that HCV core antigens can be detected with a high sensitivity without inhibition of immune reaction by subjecting an alkaline phosphatase-labeled anti-HCV core protein antibody and an HCV core protein to antigen-antibody reaction in an aqueous solution containing a salt at a high concentration, as is different from the case where an anti-HCV core protein antibody labeled with a substance other than alkaline phosphatase is used. The present invention has been completed based on this finding.

The present invention relates to the following (1)-(29).
(1) An immunoassay for detecting or determining HCV core antigens in a sample which comprises subjecting an alkaline phosphatase-labeled anti-HCV core protein antibody and an HCV core antigen in the sample to antigen-antibody reaction in an aqueous solution containing a salt at a high concentration to form an antigen-antibody complex, and measuring the alkaline phosphatase activity of the formed antigen-antibody complex.
(2) The immunoassay according to (1) wherein the salt is a halogenated alkali metal salt.
(3) The immunoassay according to (1) wherein the salt is sodium chloride.
(4) The immunoassay according to any of (1)-(3) wherein the concentration of the salt is 0.5 M to saturation.
(5) The immunoassay according to any of (1)-(4) wherein the anti-HCV core protein antibody is an antibody which recognizes a sequence of at least 5 contiguous amino acid residues in the amino acid sequence at positions 11-50 from the N-terminus of an HCV core protein.
(6) The immunoassay according to any of (1)-(4) wherein the anti-HCV core protein antibody is selected from the group consisting of KTM-145, KTM-153, KTM-157, KTM-163 and KTM-167.
(7) The immunoassay according to any of (1)-(6) wherein the sample is treated with a chaotropic substance and/or an alkali agent prior to the antigen-antibody reaction.
(8) The immunoassay according to (7) wherein the chaotropic substance is a guanidine salt.
(9) The immunoassay according to (7) or (8) wherein the alkali agent is a hydroxylated alkali metal salt.
(10) The immunoassay according to any of (7)-(9) wherein the concentration of the chaotropic substance is 1 M to saturation.
(11) The immunoassay according to any of (7)-(10) wherein the treatment with the alkali agent is carried out at 45-55°C.
(12) A reagent for the detection or determination of HCV core antigens comprising an alkaline phosphatase-labeled anti-HCV core protein antibody, a salt at a concentration of 0.5 M to saturation and a reagent for the measurement of alkaline phosphatase activity.
(13) The reagent according to (12) wherein the salt is a halogenated alkali metal salt.
(14) The reagent according to (12) or (13) wherein the salt is sodium chloride.
(15) The reagent according to any of (12)-(14) wherein the anti-HCV core protein antibody is an antibody which recognizes a sequence of at least 5 contiguous amino acid residues in the amino acid sequence at positions 11-50 from the N-terminus of an HCV core protein.
(16) The reagent according to any of (12)-(14) wherein the anti-HCV core protein antibody is selected from the group consisting of KTM-145, KTM-153, KTM-157, KTM-163 and KTM-167.
(17) A reagent kit for the detection or determination of HCV core antigens comprising the reagent according to any of (12)-(16) and a sample-treating reagent comprising a chaotropic substance and/or an alkali agent.
(18) The kit according to (17) wherein the chaotropic substance is a guanidine salt.
(19) The kit according to (17) or (18) wherein the alkali agent is a hydroxylated alkali metal salt.
(20) The kit according to any of (17)-(19) wherein the concentration of the chaotropic substance is 1 M to saturation.
(21) A monoclonal antibody which recognizes a sequence of at least 5 contiguous amino acid residues in the amino acid sequence at positions 11-50 from the N-terminus of an HCV core protein.
(22) A monoclonal antibody which recognizes the amino acid sequence at positions 41-50 from the N-terminus of an HCV core protein.
(23) The monoclonal antibody according to (22) which is produced by hybridoma KTM-145 (FERM BP-6838).
(24) A monoclonal antibody which recognizes the amino acid sequence at positions 11-30 from the N-terminus of an HCV core protein.
(25) The monoclonal antibody according to (24) which is produced by hybridoma KTM-153 (FERM BP-6839).
(26) A monoclonal antibody which recognizes the amino acid sequence at positions 31-50 from the N-terminus of an HCV core protein.
(27) The monoclonal antibody according to (26) which is produced by hybridoma KTM-157 (FERM BP-6840).
(28) A monoclonal antibody which recognizes the amino acid sequence at positions 21-40 from the N-terminus of an HCV core protein.
(29) The monoclonal antibody according to (28) which is produced by hybridoma KTM-163 (FERM BP-6841) or hybridoma KTM-167 (FERM BP-6842).

### Detailed Description of the Invention

Alkaline phosphatase of any origin may be used for preparing the alkaline phosphatase-labeled anti-HCV core protein antibodies of the present invention. For example, alkaline phosphatase derived from microorganisms such as Escherichia coli and animals such as cow can be used.

The anti-HCV core protein antibodies used for preparing the alkaline phosphatase-labeled anti-HCV core protein antibodies of the present invention refer to antibodies which react with the HCV core proteins described in Japanese Published Unexamined Patent Application No. 145194/95 and Hepatology, 16, 886 (1992). The HCV core proteins can be prepared using the recombinant DNA techniques described in Japanese Published Unexamined Patent Applications Nos. 145194/95 and 29427/96 and Hepatology, 16, 886 (1992). The antibodies can be produced by using the above recombinant HCV core proteins as immunogens according to a known method for producing a monoclonal antibody, for example, the method described in A Manual of Experiments on Monoclonal Antibodies (Sakuji Toyama edit., Kodansha).

The above-mentioned immunogen is administered to an animal such as mouse, rat, hamster, rabbit, guinea pig, goat, sheep or fowl, preferably mouse or rat, for immunization to produce the monoclonal antibodies.

Immunization can be carried out according to a known method [e.g., Saido and Toyoshima, Shin Seikagaku Jikken Koza (New Lectures on Experiments in Biochemistry), 1, 389 (1990) Tokyo Kagaku Dojin]. For example, an immunogen is emulsified with complete or incomplete Freund's adjuvant, and the resulting emulsion is administered to an animal intraperitoneally, subcutaneously or intramuscularly. Administration is made twice or more, preferably 2-4 times at regular intervals of 7-30 days, preferably 12-16 days to complete immunization.

Antibody-producing cells can be obtained from the spleen, lymph nodes, peripheral blood, etc. of an immunized animal. Antibody-producing cells can also be obtained by the so-called "in vitro immunization", that is, by directly immunizing cells responsible for antibody production which have been collected from the spleen, lymph nodes, peripheral blood, etc. of an unimmunized animal [Arai and Ohta, Experimental Medicine, 6, 43 (1988)].

There is no specific restriction as to the myeloma cells to be used for fusion with antibody-producing cells, but it is preferred to use cell lines derived from the same species of animal as the one from which the antibody-producing cells are derived. It is also preferred to use myeloma cells having specific drug markers in order to efficiently select cells which have been appropriately fused. For example, 8-azaguanine-resistant myeloma cells are preferably used because they can not grow in a medium containing hypoxanthine, aminopterin and thymidine (hereinafter referred to as HAT medium), but fused cells resulting from fusion of these myeloma cells and normal cells can grow in HAT medium and thus can be distinguished from unfused myeloma cells. Specific examples are P3 x 63-Ag. 8. 653 [J. Immunol., 123, 1548 (1979)], P3 x 63-Ag. 8. U1 [Curr. Topics. Microbiol. Immunol., 81, 1 (1978)] (hereinafter abbreviated as P3U1) and Sp/O-Ag14 [Nature, 276, 269 (1978)].

Cell fusion can be carried out by the method developed by Köhler and Milstein [Nature, 256, 495 (1975)], and its various modifications. In a method generally employed, antibody-producing cells and myeloma cells are mixed in a ratio of 10-3:1 and treated with 30-50% polyethylene glycol (average molecular weight: 1,500-6,000) as a fusing agent. Fusion can also be carried out by electroporation [Ohkouchi, et al., Experimental Medicine, 6, 50 (1988)].

The cells which have been subjected to the cell fusion treatment are suspended in a selective medium (e.g., HAT medium) and cultured in a culture vessel favorable for selecting the desired cells such as a 96-well culture plate to selectively grow fused cells. The cells which produce antibodies to an HCV core protein are selected from the thus grown fused cells by detecting the presence of desired antibodies in the culture supernatant of fused cells by a method such as enzyme immunoassay or radioimmunoassay. From the selected cells are obtained single cell colonies by the limiting dilution method, the soft agar culture method or the like to establish HCV core protein- specific monoclonal antibody-producing hybridoma cell lines.

The obtained hybridomas are cultured in an appropriate medium, or are intraperitoneally transplanted into an animal and allowed to grow in its ascites. The monoclonal antibodies can be obtained from the collected culture or ascites. The antibodies in the culture or ascites can be used after purification, if necessary. Purification can be carried out by various methods, e.g., salting-out fractionation using ammonium sulfate, ion exchange chromatography, gel filtration column chromatography, affinity column chromatography using protein A or protein G, and affinity column chromatography using antigen-immobilized gel, and combinations thereof. The antibodies useful in the present invention include antibody fragments such as Fab, Fab' and F(ab)₂ obtained by subjecting the antibodies obtained as above to treatment with an enzyme such as pepsin and/or reduction.

The preferred antibodies are those which recognize a sequence of at least 5 contiguous amino acid residues in the amino acid sequence at positions 11-50 from the N-terminus of an HCV core protein.

Specific examples of the hybridomas which produce the monoclonal antibodies of the present invention are hybridoma KTM-145, hybridoma KTM-153, hybridoma KTM-157, hybridoma KTM-163 and hybridoma KTM-167.

Hybridoma KTM-145, hybridoma KTM-153, hybridoma KTM-157, hybridoma KTM-163 and hybridoma KTM-167 were deposited with the National Institute of Bioscience and Human-Technology, Agency of Industrial Science and Technology, Ministry of International Trade and Industry, 1-3, Higashi 1-chome, Tsukuba-shi, Ibaraki-ken, 305-8566 Japan on August 12, 1999 with accession numbers FERM BP-6838, FERM BP-6839, FERM BP-6840, FERM BP-6841 and FERM BP-6842, respectively. The monoclonal antibodies produced by hybridoma KTM-145, hybridoma KTM-153, hybridoma KTM-157, hybridoma KTM-163 and hybridoma KTM-167 are hereinafter referred to merely as KTM-145, KTM-153, KTM-157, KTM-163 and KTM-167, respectively.

The alkaline phosphatase-labeled anti-HCV core protein antibodies of the present invention are obtained by binding the anti-HCV core protein antibodies and the alkaline phosphatase by covalent bonds according to methods such as the glutaraldehyde method, the periodic acid method, the maleimide method, the pyridyl disulfide method and a method using a known cross-linking agent (see, e.g., Eiji Ishikawa, Enzyme Immunoassay, Igaku Shoin).

In one embodiment, the antibody sulfhydrylated with iminothiolane or the like is mixed with the alkaline phosphatase maleimidated with SMCC (succinimidyl 4-[N-maleimidomethyl]-cyclohexane-1-carboxylate).

As used herein, an "aqueous solution containing a salt at a high concentration" refers to an aqueous solution containing an inorganic salt in an amount of 0.5 M or more. Suitable inorganic salts include halogenated alkali metal salts, e.g., sodium chloride, potassium chloride and lithium chloride. The concentration of the inorganic salt is preferably 0.5 M to saturation, more preferably 1-3 M. If necessary, the aqueous solution containing a salt at a high concentration may comprise a buffer, a salt other than sodium chloride, a surfactant, a serum, a preservative, an agent inhibiting non-specific adsorption in immune reactions, etc.

Any buffer may be used in the above solution. A suitable buffer is Tris buffer. Examples of the surfactant are Triton X-100, Triton X-705 and Tween 20. An example of the preservative is sodium azide. An example of the serum is a normal mouse serum. Examples of the salt other than sodium chloride are zinc chloride and magnesium chloride. Examples of the agent inhibiting non-specific adsorption in immune reactions are proteins such as bovine serum albumin and casein, and a commercial product Block Ace (Dainippon Pharmaceutical Co., Ltd.).

Test samples which can be assayed by the method of the present invention include whole blood, serum, plasma, urea and lymph.

The samples to be assayed for the presence of HCV antigens may be used as such or after concentration using a virus-fractionating agent. Examples of the fractionating agent are reagents for density gradient such as a polyethylene glycol (PEG) reagent and Percoll. The molecular weight and the concentration of PEG are not specifically restricted, but the molecular weight is preferably 1,000-20,000 and the concentration is preferably in the range of 3-10%.

It is preferred to treat a sample with reagents having the protein-denaturating activity, the protein-solubulizing activity, and the protein-dispersing activity. Examples of the reagents are chaotropic substances, alkali agents, acidic agents and surfactants. Preferred are chaotropic substances and alkali agents.

Examples of the chaotropic substances are urea, guanidine hydrochloride and guanidium thiocyanate. Preferred are urea and guanidine hydrochloride. The concentration of guanidine hydrochloride is preferably 1-8 M.

As the alkali agent, any alkali agent can be employed that shows a pH of 10 or more in an aqueous solution. Examples of suitable alkali agents are hydroxylated alkali metal salts. Preferred is a sodium hydroxide solution.

Examples of the acidic agents are hydrochloric acid, sulfuric acid, acetic acid, trifluoroacetic acid and trichloroacetic acid.

Suitable surfactants include nonionic surfactants, anionic surfactants, cationic surfactants and amphoteric surfactants.

It is preferred to treat a sample with both a chaotropic substance and an alkali agent. The treatment with an alkali agent is preferably carried out at a temperature of 45-55°C.

A sample treated with an acidic agent or an alkali agent is subjected to antigen-antibody reaction preferably after neutralization. Neutralization is carried out by using an alkali agent in the case of a sample treated with an acidic agent and using an acidic agent in the case of a sample treated with an alkali agent, and in both cases a solution having a high buffer capacity may also be used.

There is no specific restriction as to the methodology for the immunoassay of the present invention, and methods widely employed in the art such as sandwich immunoassay can be used.

Specifically, the immunoassay of the present invention can be carried out by sandwich immunoassays such as the known 1 step method, delay 1 step method and 2 step method using an antibody bound to a solid phase support and an alkaline phosphatase-labeled anti-HCV core protein antibody. Preferred is the 2 step method, which comprises (1) incubating an antibody-bound solid phase and a sample containing antigens in an aqueous solution, followed by washing of the solid phase with a washing solution, (2) adding an aqueous solution containing a labeled antibody and a high concentration of salt thereto, followed by further incubation and washing, and (3) measuring the alkaline phosphatase activity of an antigen-antibody complex formed on the solid phase.

Various solid phase supports used in usual immunoassays can be used in the above method. Examples of useful solid phase supports are plastic test tubes, microplate wells, glass beads, plastic beads, various membranes and magnetic particles (see, e.g., Eiji Ishikawa, Enzyme Immunoassay, Igaku Shoin). Examples of the antibodies to be bound to a solid phase support are the above-described anti-HCV core protein monoclonal antibodies, and it is preferred to use an antibody which recognizes an epitope different from that recognized by an alkaline phosphatase-labeled antibody. Binding of an antibody to a solid phase support can be carried out by known methods such as physical adsorption and chemical binding.

The alkaline phosphatase activity can be measured by conventional methods using a chromogen, a fluorescent reagent, a luminescent reagent, etc. (see, e.g., Bunji Maruo, Enzyme Handbook, Asakura Shoten). Any chromogens known in the art can be used, and these include enzyme cycling reaction reagents capable of sensitive detection, specifically, an enzyme cycling reaction reagent using an enzyme which catalyzes the cycling oxidation-reduction of coenzyme NAD (AmpliQ, Dako), etc. Examples of the fluorescent reagent are 4-methylumbelliferone derivatives and 2-hydroxybiphenyl derivatives.

Examples of the luminescent reagent are derivatives having the dioxetane structure, specifically, AMPPD [3-(2'-spiroadamantane)-4-methoxy-4-(3''-phosphoryloxy)phenyl-1,2-dioxetane], CDP-star [4-chloro-3-(methoxyspiro{1,2-dioxetane-3,2'-(5'-chloro)tricyclo[3.3.1.1^{3,7}]decane}-4-yl)phenyl disodium phosphate] and CSPD [3-(4-methoxyspiro{1,2-dioxetane-3,2'-(5'-chloro)tricyclo[3.3.1.1^{3,7}]decane}-4-yl)phenyl disodium phosphate]. Also useful are lumigen derivatives without the dioxetane structure, specifically, APS compound ([10-methyl-9(10H)-acridinylidene]phenoxymethyl disodium phosphate), etc.

The reagent for the detection or determination of HCV core antigens of the present invention comprises an alkaline phosphatase-labeled anti-HCV core protein antibody, a salt at a concentration of 0.5 M to saturation and a reagent for measurement of alkaline phosphatase activity. The reagent may further comprise an anti-HCV core protein antibody-immobilized solid phase and a standard HCV core protein. The reagent of the present invention may be in the form of a kit comprising reagents containing the above components.

The kit may further comprise a sample-concentrating reagent and a sample-treating reagent. An example of the sample-concentrating reagent is the above-mentioned fractionating agent. Examples of the sample-treating reagent are the above-mentioned reagents having the protein-denaturating activity, the protein-solubilizing activity and the protein-dispersing activity. Reagents useful for the measurement of alkaline phosphatase activity include those comprising the above-mentioned chromogen, fluorescent reagent or luminescent reagent. Examples of the anti-HCV core protein antibody are the above-mentioned monoclonal antibodies.

Certain embodiments of the present invention are illustrated in the following examples. These examples are not to be construed as limiting the scope of the invention. As the serum samples, those commercially available from Uniglobe or Boston Biomedica, Inc. (BBI) were used.

### Reference Example 1 Preparation of HCV Core Protein

A recombinant HCV core protein having 125 amino acid residues from the N-terminus of an HCV core protein was prepared and purified according to the method described in Japanese Published Unexamined Patent Application No. 29427/96.

The concentration of the purified recombinant HCV core protein was calculated using BioRad Protein Assay Kit (BioRad) and BCA Protein Assay Kit (Pierce).

### Reference Example 2 Preparation of Anti-HCV Core Protein Antibodies

The HCV core protein obtained in Reference Example 1 (25-100 µg) was emulsified with complete Freund's adjuvant, and the resulting emulsion was administered to a BALB/C mouse or an SD rat for immunization. After 2-3 weeks, 25-100 µg of the HCV core protein was emulsified with incomplete Freund's adjuvant and the resulting emulsion was administered to the animal for an additional immunization. After the rise in the antibody titer was confirmed, 25-100 µg of the HCV core protein was intravenously administered. After 3-4 days, the spleen was taken out of the animal to prepare spleen cells. Then, mouse myeloma cells (P3U1) previously cultured in RPMI-1640 medium and the spleen cells were mixed in the ratio of 1:2-1:5, followed by cell fusion using polyethylene glycol. The fused cells were suspended in HAT medium, and the resulting suspension was pipetted into a 96-well culture plate and then cultured in a CO₂-incubator at 37°C. Screening for anti-HCV core protein antibodies was carried out in the following manner.

The HCV core protein was suspended in an 8 M solution of guanidine hydrochloride or a 10 M urea solution to a concentration of 2 µg/ml, and the resulting suspension was pipetted into a 96-well ELISA plate (Nunc) in an amount of 50 µl/well and allowed to stand at 4°C overnight to adsorb the HCV core protein on the plate. After blocking with a phosphate-buffered saline (hereinafter abbreviated as a PBS solution) containing 1% bovine serum albumin (hereinafter abbreviated as BSA), 50 µl of the culture supernatant of the fused cells was put into each well, followed by reaction at room temperature for one hour. After washing, peroxidase-labeled anti-mouse IgG antibody was added to the wells, followed by reaction at room temperature for one hour. After washing, substrate ABTS was added to the wells, and the wells where coloring was observed were selected to obtain hybridomas KTM-145, KTM-153, KTM-157, KTM-163 and KTM-167 which produce antibodies recognizing the HCV core protein. The hybridomas were each transplanted into the abdomen of a mouse treated with pristane or the like to obtain monoclonal antibodies produced in the ascites. The obtained monoclonal antibodies were purified according to a conventional method using a protein A column (Sakuji Toyama edit., A Manual of Experiments on Monoclonal Antibodies, Kodansha). The obtained antibodies were named KTM-145, KTM-153, KTM-157, KTM-163 and KTM-167, respectively.

### Reference Example 3 Reaction Specificity of the Monoclonal Antibodies

The reaction specificity of the monoclonal antibodies obtained in Reference Example 2 was confirmed using an HCV core protein- or E. coli lysate-adsorbed 96-well microtiter plate. As a result, it was confirmed that KTM-145, KTM-153, KTM-157, KTM-163 and KTM-167 reacted specifically with the HCV core protein. It was also confirmed that they showed the same reaction by Western blotting. Further, epitope analyses were conducted using partial peptides of the HCV core protein, whereby it was confirmed that KTM-145 recognized the sequence at positions 41-50 from the N-terminus of the HCV core protein, KTM-153 recognized that at positions 11-30, KTM-157 recognized that at positions 31-50, and KTM-163 and KTM-167 recognized that at positions 21-40.

### Reference Example 4 Preparation of Alkaline Phosphatase-Labeled Monoclonal Antibodies

Each of the monoclonal antibodies obtained in Reference Example 2 and alkaline phosphatase (Boehringer Mannheim) were bound by maleimidation according to a conventional method (Eiji Ishikawa, Enzyme Immunoassay, Igaku Shoin). The monoclonal antibody (5 mg) was dialyzed against 50 mM Tris buffer (pH 8) and sulfhydrylated using iminothiolane. Free iminothiolane was removed from the iminothiolanated antibody by using Sephadex G-25 column (Pharmacia). The alkaline phosphatase was maleimidated using a maleimidating reagent SMCC (Pierce) and free SMCC was removed therefrom by using G-25 column.

The above sulfhydrylated monoclonal antibody and maleimidated alkaline phosphatase were mixed and subjected to reaction at 4°C overnight. After the completion of reaction, the formed alkaline phosphatase-labeled monoclonal antibody was purified by using Sephacryl S-200 column (Pharmacia).

The purified alkaline phosphatase-labeled antibody was diluted to a fixed concentration with a labeled antibody diluent [an aqueous solution comprising 50 mM Tris buffer (pH 7.6), 10 mM magnesium chloride, 0.1 mM zinc chloride and 20% Block Ace (Dainippon Pharmaceutical Co., Ltd.)] for use in enzyme-linked immunosorbent assay (ELISA).

### Reference Example 5 Preparation of Solid Phase

Each of the monoclonal antibodies obtained in Reference Example 2 was diluted with PBS containing 0.1% sodium azide to a final concentration of 5 µg/ml. The dilution of the antibody was put into wells of a 96-well microtiter plate for solid phase in an amount of 200 µl per well and allowed to stand at 4°C overnight. After washing with PBS, 300 µl of PBS containing 0.1% BSA was added to each well, and the plate was allowed to stand at 4°C overnight for blocking.

### Reference Example 6 Preparation of Peroxidase-labeled Monoclonal Antibodies

Each of the monoclonal antibodies obtained in Reference Example 2 and peroxidase (Toyobo Co., Ltd.) were bound by maleimidation according to a conventional method. The monoclonal antibody (5 mg) was dialyzed against 50 mM Tris buffer (pH 8) and sulfhydrylated using iminothiolane. Free iminothiolane was removed from the iminothiolanated antibody by using Sephadex G-25 column (Pharmacia). The peroxidase was maleimidated using a maleimidating reagent SMCC (Pierce) and free SMCC was removed therefrom by using G-25 column.

The above sulfhydrylated monoclonal antibody and maleimidated peroxidase were mixed and subjected to reaction at 4°C overnight. The obtained peroxidase-labeled monoclonal antibody was purified by using Sephacryl S-200 column (Pharmacia).

The purified labeled antibody was diluted to a fixed concentration with a labeled antibody diluent [an aqueous solution comprising 50 mM Tris buffer (pH 7.6), 10 mM magnesium chloride, 0.1 mM zinc chloride and 20% Block Ace (Dainippon Pharmaceutical Co., Ltd.)] for use in ELISA.

### Example 1 Determination of Recombinant HCV Core Protein

The recombinant HCV core protein obtained in Reference Example 1 was diluted with PBS containing 0.1% BSA to concentrations of 0 and 100 pg/ml.

Each of the diluted solutions of the recombinant HCV core protein was put into wells of the microplate prepared in Reference Example 5 in an amount of 200 µl per well, followed by reaction at room temperature for one hour. After washing with a washing solution (accompanied by AMPAK sensitivity-enhancing reagent, Dako), the labeled antibody obtained in Reference Example 4 was added to the wells. The labeled antibody was diluted with a solution comprising 50 mM Tris buffer (pH 7.6), 10 mM magnesium chloride, 0.1 mM zinc chloride, 20% Block Ace (Dainippon Pharmaceutical Co., Ltd.) and 0-3 M sodium chloride, and the resulting dilution was put into the wells in an amount of 200 µl per well. After reaction with stirring at room temperature for one hour, the wells were washed with a washing solution, and 200 µl of a coloring reagent [an enzyme cycling reaction reagent (Dako)] was added to each well, followed by reaction with stirring at room temperature for 20 minutes. To each well was added 100 µl of a reaction terminator attached to the above reagent and the absorbance was measured at the main wavelength of 490 nm and the sub wavelength of 660 nm. In this test, KTM-145 was used for the solid phase and KTM-163 for the alkaline phosphatase labeling.

The results are shown in Table 1. In Table 1, the figures for each concentration of antigen indicate the absorbances (Abs).

**Table 1**

| Concentration of sodium chloride (M) | Concentration of antigen (pg/ml) | | S/N ratio |
|---|---|---|---|
| | 0 | 100 | |
| 0 | 0.146 | 0.226 | 1.55 |
| 0.25 | 0.145 | 0.317 | 2.18 |
| 0.5 | 0.146 | 0.453 | 3.11 |
| 0.75 | 0.144 | 0.583 | 4.06 |
| 1.0 | 0.150 | 0.666 | 4.45 |
| 1.5 | 0.151 | 0.818 | 5.43 |
| 2.0 | 0.152 | 0.925 | 6.10 |
| 3.0 | 0.195 | 1.032 | 5.29 |

The raise in the concentration of sodium chloride in the dilution of the labeled antibody little affected the absorbance in the test group with no antigen (0 pg/ml); however, it caused the increase in the absorbance in the test group with antigen (100 pg/ml) . In the table, the S/N ratio indicates the ratio of the absorbance in the group with antigen (100 pg/ml) to that in the group with no antigen (0 pg/ml). It has been found that addition of sodium chloride increases the S/N ratio and enables the detection of antigen with a higher sensitivity. That is, the above result indicates that application of the method of the present invention enables accurate judgment of a sample which is incapable of being judged antigen-positive in spite of the presence of antigen because of slight increase in absorbance compared with a sample with no antigen in the absence of sodium chloride.

### Example 2 Determination of Recombinant HCV Core Protein

The recombinant HCV core protein obtained in Reference Example 1 was diluted with PBS containing 0.1% BSA to concentrations ranging from 0 to 20 pg/ml.

Each of the diluted solutions of the recombinant HCV core protein was put into wells of the microplate prepared in Reference Example 5 in an amount of 200 µl per well, followed by reaction at room temperature for one hour. After washing with a washing solution (accompanied by AMPAK sensitivity-enhancing reagent, Dako), the labeled antibody obtained in Reference Example 4 was added to the wells. The labeled antibody was diluted with a solution comprising 50 mM Tris buffer (pH 7.6), 10 mM magnesium chloride, 0.1 mM zinc chloride, 20% Block Ace (Dainippon Pharmaceutical Co., Ltd.) and 0-2 M sodium chloride, and the resulting dilution was put into the wells in an amount of 200 µl per well. After reaction with stirring at room temperature for one hour, the wells were washed with a washing solution, and 200 µl of a coloring reagent [an enzyme cycling reaction reagent (Dako)] was added to each well, followed by reaction with stirring at room temperature for 40 minutes. To each well was added 100 µl of a reaction terminator attached to the above reagent and the absorbance was measured at the main wavelength of 490 nm and the sub wavelength of 660 nm. In this test, KTM-145 was used for the solid phase and KTM-163 for the alkaline phosphatase labeling.

The results are shown in Table 2. In Table 2, the figures for each concentration of antigen indicate the absorbances (Abs).

**Table 2**

| Concentration of sodium chloride (M) | Concentration of antigen (pg/ml) | | | |
|---|---|---|---|---|
| | 0 | 5 | 10 | 20 |
| 0 | 0.178 | 0.345 | 0.448 | 0.747 |
| 0.25 | 0.182 | 0.371 | 0.539 | 0.924 |
| 0.5 | 0.180 | 0.448 | 0.691 | 1.284 |
| 0.75 | 0.239 | 0.618 | 0.959 | 1.676 |
| 1.0 | 0.147 | 0.595 | 1.059 | 2.061 |
| 1.5 | 0.162 | 0.725 | 1.291 | 2.477 |
| 2.0 | 0.166 | 0.754 | 1.389 | 2.557 |

The raise in the concentration of sodium chloride in the dilution of the labeled antibody little affected the absorbance in the test group with no antigen; however, it caused the increase in the absorbance in the test group with antigen. This result indicates that application of the method of the present invention enables accurate judgment of a sample which is incapable of being judged antigen-positive in spite of the presence of antigen because of slight increase in absorbance compared with a sample with no antigen in the absence of sodium chloride.

### Example 3 Detection Limit

The recombinant HCV core protein obtained in Reference Example 1 was diluted with PBS containing 0.1% BSA to concentrations ranging from 0 to 10 pg/ml.

Each of the diluted solutions of the recombinant HCV core protein was put into wells of the microplate prepared in Reference Example 5 in an amount of 200 µl per well, followed by reaction at room temperature for one hour. After washing with a washing solution (accompanied by AMPAK sensitivity-enhancing reagent, Dako), the labeled antibody obtained in Reference Example 4 was added to the wells. The labeled antibody was diluted with a solution comprising 50 mM Tris buffer (pH 7.6), 10 mM magnesium chloride, 0.1 mM zinc chloride, 20% Block Ace (Dainippon Pharmaceutical Co., Ltd.) and 0 or 1 M sodium chloride, and the resulting dilution was put into the wells in an amount of 200 µl per well. After reaction with stirring at room temperature for one hour, the wells were washed with a washing solution, and 100 µl of a coloring reagent [an enzyme cycling reaction reagent (Dako)] was added to each well, followed by reaction with stirring at room temperature for 30 minutes. To each well was added 100 µl of a reaction terminator attached to the above reagent and the absorbance was measured at the main wavelength of 490 nm and the sub wavelength of 660 nm. In this test, KTM-145 was used for the solid phase and KTM-163 for the alkaline phosphatase labeling.

The results are shown in Table 3. In Table 3, the figures for each concentration of salt indicate the absorbances (Abs).

**Table 3**

| Concentration of antigen (pg/ml) | Concentration of salt (M) | |
|---|---|---|
| | 0 | 1 |
| 0 | 0.100 | 0.067 |
| 0.125 | Not tested | 0.080 |
| 0.25 | Not tested | 0.096 |
| 0.5 | Not tested | 0.120 |
| 1 | 0.114 | 0.178 |
| 2 | Not tested | 0.299 |
| 5 | 0.172 | 0.686 |
| 10 | Not tested | 1.325 |

The raise in the concentration of sodium chloride in the dilution of the labeled antibody enabled the detection of the antigen at extremely low concentrations. As shown in Table 3, when sodium chloride was not added, there was observed a difference in the absorbance of the order of 10 mAbs between the solution containing no antigen and the solution containing 1 pg/ml antigen, while there was a difference in the absorbance of the order of 10 mAbs between the solution containing no antigen and the solution containing 0.125 pg/ml antigen when 1 M sodium chloride was added. It indicates that the addition of sodium chloride enhanced the sensitivity for detection.

### Comparative Example 1

The recombinant HCV core protein obtained in Reference Example 1 was diluted with PBS containing 0.1% BSA to concentrations of 0, 10, 100 and 1000 pg/ml.

Each of the diluted solutions of the recombinant HCV core protein was put into wells of the microplate prepared in Reference Example 5 in an amount of 200 µl per well, followed by reaction at room temperature for one hour. After washing with a washing solution (accompanied by AMPAK sensitivity-enhancing reagent, Dako), the peroxidase-labeled antibody obtained in Reference Example 6 was added to the wells. The labeled antibody was diluted with a solution comprising 50 mM Tris buffer (pH 7.2), 0.1% BSA and 0-4 M sodium chloride, and the resulting dilution was put into the wells in an amount of 200 µl per well. After reaction with stirring at room temperature for one hour, the wells were washed with a washing solution, and 200 µl of a coloring reagent TMB (tetramethylbenzidine, Intergen Co.) was added to each well, followed by reaction with stirring at room temperature for 30 minutes. To each well was added 100 µl of 1 N sulfuric acid to stop the coloring reaction and the absorbance was measured at the main wavelength of 450 nm and the sub wavelength of 660 nm. In this test, KTM-145 was used for the solid phase and KTM-163 for the peroxidase labeling.

The results are shown in Table 4. In Table 4, the figures for each concentration of antigen indicate the absorbances (Abs).

**Table 4**

| Concentration of sodium chloride (M) | Concentration of antigen (pg/ml) | | | |
|---|---|---|---|---|
| | 0 | 10 | 100 | 1000 |
| 0 | 0.125 | 0.136 | 0.144 | 0.296 |
| 0.1 | 0.166 | 0.188 | 0.202 | 0.479 |
| 0.2 | 0.191 | 0.211 | 0.251 | 0.606 |
| 0.5 | 0.217 | 0.234 | 0.310 | 0.893 |
| 1.0 | 0.234 | 0.284 | 0.372 | 1.076 |
| 1.5 | 0.283 | 0.331 | 0.388 | 1.050 |
| 2.0 | 0.403 | 0.387 | 0.411 | 1.141 |
| 3.0 | 0.616 | 0.588 | 0.624 | 1.138 |
| 4.0 | 0.898 | 0.839 | 0.915 | 1.293 |

When the peroxidase-labeled antibody was used, the addition of sodium chloride caused lowering of the reaction specificity in the reaction system with no antigen. In the reaction systems with antigen, the raise in the concentration of sodium chloride did not improve the reaction specificity.

### Example 4 Assay of Human Serum Samples

Human sera were treated according to the method described in Japanese Published Unexamined Patent Application No. 29427/96. That is, 50 µl of 20% PEG4000 was added to 200 µl of a serum sample, and the resulting mixture was allowed to stand on ice for one hour. After centrifugation at 4000 x g at 4°C for 5 minutes, the precipitate was suspended in 50 µl of 50 mM Tris buffer (pH 8.0), and 50 µl of a 0.5 M sodium hydroxide solution was added thereto, followed by treatment at 37°C for 30 minutes. Then, the mixture was neutralized with 50 µl of a 0.5 M sodium hydrogenphosphate solution containing 0.3% Triton X-100. The resulting mixture was diluted three-fold with 300 µl of a 0.1% BSA-PBS solution to prepare a solution for assay. As the human serum samples, anti-HCV antibody-positive samples (Uni-2 and 0401) and a normal sample as a control were employed and tested in the same manner as in Example 1 for measurement of the absorbance (Abs). The results are shown in Table 5.

**Table 5**

| Concentration of sodium chloride (M) | Normal sample | Sample Uni-2 | | Sample 0401 | |
|---|---|---|---|---|---|
| | Absorbance | Absorbance | S/N ratio | Absorbance | S/N ratio |
| 0 | 0.149 | 0.166 | 1.11 | 0.201 | 1.35 |
| 0.25 | 0.148 | 0.189 | 1.27 | 0.269 | 1.81 |
| 0.50 | 0.146 | 0.237 | 1.63 | 0.385 | 2.65 |
| 0.75 | 0.149 | 0.287 | 1.93 | 0.464 | 3.12 |
| 1.00 | 0.152 | 0.314 | 2.07 | 0.542 | 3.57 |
| 1.50 | 0.156 | 0.360 | 2.30 | 0.665 | 4.26 |
| 2.00 | 0.164 | 0.397 | 2.42 | 0.726 | 4.43 |
| 3.00 | 0.241 | 0.526 | 2.19 | 0.953 | 3.96 |

As shown in Table 5, when the normal serum sample was used, the absorbance was increased only a little by raising the concentration of sodium chloride in the dilution of the labeled antibody. On the other hand, when the anti-HCV antibody-positive serum samples were used, the absorbance was increased by raising the concentration of sodium chloride in the dilution of the labeled antibody. The result indicates that samples containing lower levels of antigen can be correctly judged antigen-positive by the use of the present method. It is also clear from the S/N ratio that the sensitivity is remarkably enhanced when the concentration of sodium chloride is 0.5-3 M, specifically 1-3 M.

### Example 5 Treatment of Samples with Guanidine Hydrochloride and Alkali

To 200 µl of a serum sample was added 50 µl of 20% PEG4000, and the resulting mixture was allowed to stand on ice for one hour. After centrifugation at 4000 x g at 4°C for 5 minutes, the precipitate was suspended in 50 µl of 50 mM Tris buffer (pH 8.0) containing 0-8 M guanidine hydrochloride for denaturation. To the resulting suspension was added 50 µl of a 0.5 M sodium hydroxide solution, followed by treatment at 37°C for 30 minutes, and the resulting mixture was neutralized with a 0.5 M sodium hydrogenphosphate solution. Then, the resulting mixture was diluted three-fold with a 0.1% BSA-PBS solution to prepare a solution for assay. Assay was carried out in the same manner as in Example 4, except that the concentration of sodium chloride was 1 M. As the human samples, a normal human serum sample and 3 anti-HCV antibody-positive serum samples (U-19, U-21 and U-29) were used. The results are shown in Table 6. In Table 6, the figures for each sample indicate the absorbances (Abs).

**Table 6**

| Guanidine hydrochloride (M) | Normal sample | U-19 | U-21 | U-29 |
|---|---|---|---|---|
| 0 | 0.129 | 0.890 | 0.350 | 0.320 |
| 2 | 0.129 | 1.020 | 0.503 | 0.499 |
| 4 | 0.059 | 1.358 | 0.816 | 0.631 |
| 6 | 0.045 | 1.982 | 1.226 | 0.912 |
| 8 | 0.052 | 2.614 | 1.798 | 1.268 |

As shown in Table 6, the absorbance increased depending upon the concentration of guanidine hydrochloride.

### Example 6

The same procedure as in Example 5 was repeated using urea and guanidine hydrochloride. The concentration of urea was 10 M and that of guanidine hydrochloride was 8 M. As the samples, a normal human serum sample and 4 anti-HCV antibody-positive serum samples (104, 106, 187 and 197) were used. The results are shown in Table 7. In Table 7, the figures for each reagent indicate the absorbances (Abs).

**Table 7**

| Sample | Guanidine hydrochloride | Urea |
|---|---|---|
| Normal | 0.044 | 0.041 |
| 104 | 0.078 | 0.059 |
| 106 | 0.654 | 0.416 |
| 187 | 0.429 | 0.252 |
| 197 | 0.165 | 0.042 |

As shown in Table 7, when the samples were treated with urea, there was no significant difference in the absorbance between the normal human serum and samples 104 and 197, and these two samples could not be judged positive. However, the treatment with guanidine hydrochloride enabled the judgement of these samples as positive.

### Example 7 Experiment on the Temperature for Alkali Treatment

The same procedure as in Example 6 was repeated, except that the temperature for alkali treatment was varied. As the serum samples, samples 6, 9 and 138 were used. The alkali treatment was carried out at 37°C, 50°C, 70°C and 100°C. The results are shown in Table 8. In Table 8, the figures for each sample indicate the absorbances (Abs).

**Table 8**

| Temperature (°C) | Normal sample | Sample 6 | Sample 9 | Sample 138 |
|---|---|---|---|---|
| 37 | 0.065 | 0.438 | 0.198 | 0.405 |
| 50 | 0.064 | 0.482 | 0.218 | 0.474 |
| 70 | 0.061 | 0.302 | 0.151 | 0.345 |
| 100 | 0.058 | 0.089 | 0.076 | 0.102 |

As shown in Table 8, a preferred temperature for the alkali treatment of a serum is around 50°C.

### Example 8 Comparison with Various Methods for Detection

Assay according to the present invention was carried out in the following manner. To 200 µl of each serum sample was added 50 µl of 20% PEG4000, and the resulting mixture was allowed to stand on ice for one hour. After centrifugation at 4000 x g at 4°C for 5 minutes, the precipitate was suspended in 50 µl of 50 mM Tris buffer (pH 8.0) containing 8 M guanidine hydrochloride for denaturation. To the resulting suspension was added 50 µl of a 0.5 M sodium hydroxide solution, followed by treatment at 50°C for 30 minutes, and the resulting mixture was neutralized with a 0.5 M sodium hydrogenphosphate solution containing 0.3% Triton X-100. Then, the resulting mixture was diluted three-fold with a 0.1% BSA-PBS solution to prepare a solution for assay. Assay was carried out in the same manner as in Example 4, except that 2 M sodium chloride was used in the dilution of the labeled antibody.

In addition, assay of the same samples was carried out using the following commercially available reagents: Immunocheck F-HCV Ag core which is an assay kit for HCV core protein (Kokusai Siyaku, hereinafter referred to merely as HCV core protein assay kit), the third generation anti-HCV antibodies which is an assay kit for anti-HCV antibodies (Ortho, hereinafter referred to merely as anti-HCV antibody assay kit) and AmpliCore HCV monitor which is an assay kit for RNA of HCV by PCR (Roche, hereinafter referred to merely as RNA assay kit) to compare the sensitivity with that of the present method.

The results are shown in Table 9. In Table 9, "-" means "not detectable" and "+" means "detectable".

**Table 9**

| Sample | The present invention | | HCV core protein assay kit | Anti-HCV antibody assay kit | RNA assay kit |
|---|---|---|---|---|---|
| | Absorbance (Abs) | Judgment | | | |
| Normal 1 | 0.059 | - | - | - | - |
| Normal 2 | 0.060 | - | - | - | - |
| Normal 3 | 0.062 | - | - | - | - |
| Normal 4 | 0.062 | - | - | - | - |
| Normal 5 | 0.061 | - | - | - | - |
| Normal 6 | 0.061 | - | - | - | - |
| U-19 | 2.973 | + | + | + | + |
| B-2 | 0.910 | + | + | + | + |
| U-16 | 0.867 | + | + | + | + |
| B-19 | 0.212 | + | + | + | + |
| T1 | 0.582 | + | + | + | Not measured |
| T2 | 0.145 | + | - | + | Not measured |
| T3 | 0.134 | + | - | + | Not measured |
| U168 | 0.117 | + | - | + | + |
| U169 | 0.149 | + | - | + | + |

As shown in Table 9, tests by the method of the present invention gave positive results on the samples which could not be judged positive by the HCV core protein assay kit (Kokusai Siyaku). The serum samples except for the normal human serum samples are anti-HCV antibody-positive and the samples tested by the RNA assay kit (Roche) were judged HCV-positive.

The sensitivity of the HCV core protein assay kit (Kokusai Siyaku) for assay of serum samples was of the order of 10⁴ virions/ml, while that of the method of the present invention was of the order of 10³ virions/ml, which was equal to the sensitivity of the RNA assay kit (Roche).

### Example 9 Combination of Antibodies

The same procedure as in Example 6 was repeated, except that the kinds of the antibodies used for the solid phase and the alkaline phosphatase-labeling were changed, a serum from an HCV-uninfected human and a serum from an HCV-infected human were used as samples and the concentration of sodium chloride in the aqueous solution to dilute the labeled antibody was 0 or 1 M. The absorbance was measured to calculate the S/N ratio in each test. The results are shown in Table 10. In Table 10, the figures for each concentration of sodium chloride indicate the S/N ratios.

**Table 10**

| Solid phase antibody | Labeled antibody | Concentration of sodium chloride (M) | |
|---|---|---|---|
| | | 0 | 1 |
| KTM-153 | KTM-145 | 3.07 | 5.46 |
| KTM-145 | KTM-153 | 1.46 | 6.48 |
| KTM-153 | KTM-157 | 2.96 | 11.17 |
| KTM-145 | KTM-167 | 7.20 | 15.61 |
| KTM-145 | KTM-163 | 7.50 | 15.30 |

As shown in Table 10, in any combination of solid phase antibody and labeled antibody, the S/N ratio was increased by addition of sodium chloride to the solution to dilute the labeled antibody. It indicates that the detection of HCV infection can be carried out with a higher sensitivity in the presence of sodium chloride.

### Example 10

Each of the sample solutions (U-14 and U-19) was sequentially diluted with a solution of normal serum to prepare samples. To 200 µl of each sequentially diluted sample was added 50 µl of 20% PEG4000, and the resulting mixture was allowed to stand on ice for one hour. After centrifugation at 4000 x g at 4°C for 5 minutes, the precipitate was suspended in 50 µl of 50 mM Tris buffer (pH 8.0) containing 8 M guanidine hydrochloride for denaturation. To the resulting suspension was added 50 µl of a 0.5 M sodium hydroxide solution, followed by treatment at 50°C for 30 minutes, and the resulting mixture was neutralized with a 0.5 M sodium hydrogenphosphate solution containing 0.3% Triton X-100. Then, the resulting mixture was diluted three-fold with a 0.1% BSA-PBS solution to prepare a solution for assay. Each diluted sample was assayed according to the method described in Example 4. The concentration of sodium chloride in the dilution of the labeled antibody was 2 M.

The calibration curve was prepared with the HCV core protein obtained in Reference Example 1 at known concentrations as a standard substance and the amount of HCV core protein was calculated from the absorbance (Abs) of each sample.

The results of the tests using sample U-14 and sample U-19 are shown in Table 11 and Table 12, respectively.

**Table 11**

| Dilution of sample | Sample U-14 | |
|---|---|---|
| | Absorbance | Concentration (pg/ml) |
| 1/128 | 0.073 | 0.14 |
| 1/64 | 0.082 | 0.26 |
| 1/32 | 0.105 | 0.56 |
| 1/16 | 0.146 | 1.11 |
| 1/8 | 0.204 | 1.86 |
| 1/4 | 0.372 | 4.08 |
| 1/2 | 0.661 | 7.89 |
| 1 | 1.219 | 15.26 |

**Table 12**

| Dilution of sample | Sample U-19 | |
|---|---|---|
| | Absorbance | Concentration (pg/ml) |
| 1/800 | 0.084 | 0.28 |
| 1/400 | 0.104 | 0.59 |
| 1/200 | 0.138 | 1.11 |
| 1/100 | 0.199 | 2.07 |
| 1/10 | 1.413 | 20.40 |

As shown in Table 11 and Table 12, the method of determination of the present invention is capable of detection of the HCV core protein to the order of 0.1 pg/ml.

### Example 11 Detection of HCV Core Antigen in a Seroconversion Panel Sample

A commercially available seroconversion panel PHV 908 (BBI) was assayed for HCV core antigen according to the method described in Example 8. The amount of HCV core antigen was expressed in terms of the S/N ratio regarding the absorbance of a normal human sample as 1.0.

The above panel was also assayed with the anti-HCV antibody assay kit (Ortho). The antibody titer was expressed in terms of the cut-off index (S/CO) and samples having the antibody titer of 1.0 or more were judged to be positive.

The results are shown in Table 13.

**Table 13**

| Sample No. | Days after the start of observation | The method of the present invention (S/N ratio) | Anti-HCV antibody assay kit (S/CO) |
|---|---|---|---|
| 01 | 0 | 6.5 | 0 |
| 02 | 3 | 6.4 | 0 |
| 03 | 5 | 4.8 | 0 |
| 04 | 11 | 7.1 | 0.1 |
| 05 | 13 | 7.1 | 0.3 |
| 06 | 19 | 10.9 | 1.7 |
| 07 | 25 | 11.1 | 4.9 |
| 08 | 27 | 6.9 | 4.9 |
| 09 | 32 | 4.5 | >5.0 |
| 10 | 35 | 1.5 | >5.0 |
| 11 | 41 | 1.9 | >5.0 |
| 12 | 45 | 1.3 | >5.0 |
| 13 | 48 | 6.4 | >5.0 |

As shown in Table 13, the method of the present invention was capable of detecting the core antigen before a positive result was obtained in the test for anti-HCV antibody; that is, the method is capable of detection of HCV infection in its early stage.

### Example 12 HCV Core Protein Assay Kit

A reagent kit comprised of the following reagents was prepared.
Antibody-coated plate
   (prepared in the same manner as in Reference Example 5 using KTM-145 obtained in Reference Example 2)
Sample diluent
   (an aqueous solution comprising PBS containing 0.1% BSA and 1% normal mouse serum)
Enzyme-labeled antibody
   (prepared in the same manner as in Reference Example 4 using KTM-163 obtained in Reference Example 2)
Standard antigen
   0 pg/ml
   10 pg/ml
   (prepared by dissolving the HCV core protein obtained in Reference Example 1 in a solution comprising PBS containing 0.067% BSA, 0.89 M guanidine hydrochloride, 5.56 mM Tris buffer (pH 8), 0.056 M sodium hydroxide, 0.056 M sodium dihydrogenphosphate and 0.005% Triton X-100 to a concentration of 0 or 10 pg/ml)
Labeled antibody diluent
   (comprising 50 mM Tris buffer (pH 7.6), 10 mM magnesium chloride, 0.2 mM zinc chloride, 20% Block Ace, 2 M sodium chloride, 0.1% sodium azide, 0.1% Triton X-705 and 1% normal mouse serum)
Coloring reagent
   [a coloring reagent contained in an enzyme cycling reaction reagent (AmpliQ, Dako)]
Reaction terminator
   [a reaction terminator contained in an enzyme cycling reaction reagent (AmpliQ, Dako)
Precipitant
   (20% PEG4000)
Dispersing reagent
   [8 M guanidine hydrochloride and 50 mM Tris buffer (pH 8)]
Alkali reagent
   (a 0.5 M sodium hydroxide solution)
Neutralizer
   (0.5 M sodium dihydrogenphosphate and 0.3% Triton X-100)

## Claims

1. An immunoassay for detecting or determining hepatitis C virus (HCV) core antigens in a sample which comprises subjecting an alkaline phosphatase-labeled anti-HCV core protein antibody and an HCV core antigen in the sample to antigen-antibody reaction in an aqueous solution containing a salt at a high concentration to form an antigen-antibody complex, and measuring the alkaline phosphatase activity of the formed antigen-antibody complex.

2. The immunoassay according to claim 1 wherein the salt is a halogenated alkali metal salt.

3. The immunoassay according to claim 1 wherein the salt is sodium chloride.

4. The immunoassay according to any of claims 1-3 wherein the concentration of the salt is 0.5 M to saturation.

5. The immunoassay according to any of claims 1-4 wherein the anti-HCV core protein antibody is an antibody which recognizes a sequence of at least 5 contiguous amino acid residues in the amino acid sequence at positions 11-50 from the N-terminus of an HCV core protein.

6. The immunoassay according to any of claims 1-4 wherein the anti-HCV core protein antibody is selected from the group consisting of KTM-145, KTM-153, KTM-157, KTM-163 and KTM-167.

7. The immunoassay according to any of claims 1-6 wherein the sample is treated with a chaotropic substance and/or an alkali agent prior to the antigen-antibody reaction.

8. The immunoassay according to claim 7 wherein the chaotropic substance is a guanidine salt.

9. The immunoassay according to claim 7 or 8 wherein the alkali agent is a hydroxylated alkali metal salt.

10. The immunoassay according to any of claims 7-9 wherein the concentration of the chaotropic substance is 1 M to saturation.

11. The immunoassay according to any of claims 7-10 wherein the treatment with the alkali agent is carried out at 45-55°C.

12. A reagent for the detection or determination of HCV core antigens comprising an alkaline phosphatase-labeled anti-HCV core protein antibody, a salt at a concentration of 0.5 M to saturation and a reagent for the measurement of alkaline phosphatase activity.

13. The reagent according to claim 12 wherein the salt is a halogenated alkali metal salt.

14. The reagent according to claim 12 or 13 wherein the salt is sodium chloride.

15. The reagent according to any of claims 12-14 wherein the anti-HCV core protein antibody is an antibody which recognizes a sequence of at least 5 contiguous amino acid residues in the amino acid sequence at positions 11-50 from the N-terminus of an HCV core protein.

16. The reagent according to any of claims 12-14 wherein the anti-HCV core protein antibody is selected from the group consisting of KTM-145, KTM-153, KTM-157, KTM-163 and KTM-167.

17. A reagent kit for the detection or determination of HCV core antigens comprising the reagent according to any of claims 12-16 and a sample-treating reagent comprising a chaotropic substance and/or an alkali agent.

18. The kit according to claim 17 wherein the chaotropic substance is a guanidine salt.

19. The kit according to claim 17 or 18 wherein the alkali agent is a hydroxylated alkali metal salt.

20. The kit according to any of claims 17-19 wherein the concentration of the chaotropic substance is 1 M to saturation.

21. A monoclonal antibody which recognizes a sequence of at least 5 contiguous amino acid residues in the amino acid sequence at positions 11-50 from the N-terminus of an HCV core protein.

22. A monoclonal antibody which recognizes the amino acid sequence at positions 41-50 from the N-terminus of an HCV core protein.

23. The monoclonal antibody according to claim 22 which is produced by hybridoma KTM-145 (FERM BP-6838).

24. A monoclonal antibody which recognizes the amino acid sequence at positions 11-30 from the N-terminus of an HCV core protein.

25. The monoclonal antibody according to claim 24 which is produced by hybridoma KTM-153 (FERM BP-6839).

26. A monoclonal antibody which recognizes the amino acid sequence at positions 31-50 from the N-terminus of an HCV core protein.

27. The monoclonal antibody according to claim 26 which is produced by hybridoma KTM-157 (FERM BP-6840).

28. A monoclonal antibody which recognizes the amino acid sequence at positions 21-40 from the N-terminus of an HCV core protein.

29. The monoclonal antibody according to claim 28 which is produced by hybridoma KTM-163 (FERM BP-6841) or hybridoma KTM-167 (FERM BP-6842).
